# EUROPEAN PATENT APPLICATION

(11) **EP 4 145 114 A1**
(43) Date of publication of application: **08.03.2023**
(21) Application number: 21194392.3
(22) Date of filing: 01.09.2021
(51) Int. Cl.: G01N 21/65, G01N 21/77, G01N 33/52, G01N 33/543

(54) **SAMPLE PREPARATION FOR DETECTING DRUGS IN BODY FLUIDS**

(71) Applicant: Securetec Detektions-Systeme AG, 85579 Neubiberg (DE)
(72) Inventor: KAATZ, Miriam, 81737 München (DE); RINGLSTETTER, Stefan, 82386 Huglfing (DE); WENDELGAß, Fabian, 85649 Brunnthal (DE)
(74) Representative: DTS Patent- und Rechtsanwälte Schnekenbühl und Partner mbB

(57) **Abstract**

A device (10) for pretreating a sample, comprising: a pretreatment zone (11) which is permeable for at least part of a liquid comprised by the body fluid sample, wherein the pretreatment zone comprises an organic polymer and/or an inorganic oxide or hydroxide.

## Description

The present invention relates to a device and a method for pretreating a sample, e.g. a body fluid sample isolated from a human and/or a material sample, and to the use of specific materials for pretreating a sample, e.g. a body fluid sample isolated from a human and/or a material sample for drug testing.

The ability to detect and identify trace amounts of chemicals has become increasingly important in a wide variety of scientific disciplines such as in the field of medicinal-, environmental-, food- and agroanalytics and in addition in the fields of public safety, infringement of the law and crime.

A vertical-flow paper surface enhanced Raman spectroscopy (SERS) system for therapeutic drug monitoring of flucytosine in serum was described by Berger *et al.* An optimized vertical-flow system with nitrocellulose membranes and a paper SERS sensor was used. Detection of down to 10 µg mL⁻¹ flucytosine was achieved in undiluted serum, with quantitative detection across the entire therapeutic range. Inkjet-printed surface enhanced Raman sensors were used (Adam G. Berger, Stephen M. Restaino, Ian M. White, Vertical-flow paper SERS system for therapeutic drug monitoring of flucytosine in serum, Analytica Chimica Acta, Volume 949, 2017, Pages 59-66).

Berger *et al.* in the materials section briefly mention a range of different membranes which have been purchased by the authors. These include: Whatman Grade 1 chromatography paper which was purchased as a reel from GE Healthcare (Pittsburgh, PA); Nitrocellulose membranes (0.45 µm pores) which were obtained as a reel from Bio-Rad (Hercules, CA); Durapore Hydrophilic polyvinylidene fluoride (PVDF) Membranes (0.18 µm pores, 90 mm diameter) and Durapore Hydrophobic PVDF Membranes (0.22 µm pores, 13 mm diameter) were both purchased from Millipore (Darmstadt, Germany); Polycarbonate membrane filters (5 µm pores, 25 × 80 mm) and nylon membrane filters (5 µm pores, 25 mm diameter) which were obtained from Sterlitech (Kent, WA).

In the vertical flow setup used by Berger *et al.,* the sensor was positioned with the sensor face up on a glass slide. The microporous membranes are placed on top of the nanoparticle detection zone. To align the vertical flow components, the underside of the membranes was illuminated to visualize the sensor. Sample droplets were placed on top of the uppermost microporous membrane directly over the nanoparticle region of the underlying SERS sensor. Another glass slide was placed directly on top of the membranes and the entire setup was clamped with a pony-style spring clamp to maintain contact between membranes and the sensor.

Berger *et al.* explain that utilizing nitrocellulose as the filtering membrane resulted in the highest discrimination. The authors state that other materials may not bind protein as well or may prevent the passage of flucytosine. The authors further optimized the number of nitrocellulose membranes in the vertical flow stack and the sample volume.

Berger *et al.* are strictly focused on therapeutic drug monitoring (TDM). Berger *et al.* have not tried to subject saliva to their vertical flow stack. They have not identified potentially disturbing components in saliva that might interfere with SERS. They have in particular not developed a strategy for removing such disturbing components from saliva.

Another particular field of application of analytical techniques that is important in practice is detecting illicit and pharmaceutical drugs in bodily fluids or on surfaces which are contaminated with drugs. The number of drug related emergency room visits has drastically increased in recent years including cases attributed to illicit drugs and pharmaceutical drugs. Illicit drugs include cannabis, cocaine, heroin, methamphetamines, PCP, MDMA (known as ecstasy), and LSD. Pharmaceutical drugs include prescription and over-the-counter drugs, the leading prescription drugs being oxycodone, hydrocodone, and diazepam.

Drug consumption is a large problem in nearly all societies. Consumers not only harm themselves but also others, for example, when driving a motor vehicle under the influence of drugs. In order to decrease the risk of danger to others, it is necessary to have effective ways of measuring drug consumption on location, for example at a roadside inspection.

An aspect of major importance especially for detecting drugs is the specificity, sensitivity, and rapidity of the tests which are used. Usually, it is not known which drug a person consumed as there is a wide variety of different illicit and pharmaceutical drugs. It is thus necessary to have a test that can recognize as many different analytes as possible. On the one hand there is a need for highly sensitive detection methods so as to be able to trace the presence of drugs reliably and rapidly even with small sample volumes or when using complex sample material such as saliva. On the other hand, the test formats should also have a high specificity to the substance which is to be traced in each case so as to exclude false-positive measurement results, and thus provide authoritative information as to which specific drug the tested substance is. Ideally, the device would be portable, easy to use, and relatively non-invasive. The last requirement can best be met using saliva as the sample medium.

Many different types of screening devices are available in the art such as immunoassay kits that provide drug identification. The main problem with immunological tests, however, is that they are only suitable for a limited number of analytes. They usually contain detecting reagents for the most common drugs. However, it is not possible to cover a wide range of drugs.

Another method of drug detection is based on spectroscopic measuring. In a lab, sophisticated instruments such as gas chromatographs coupled with mass spectrometers can be used to verify drug identification as well as provide quantification. However, such instruments are not transportable and cannot be used for drug detection on location.

In recent years, the potential of SERS to both identify and quantify drugs and their metabolites was investigated. This approach is based on the extreme sensitivity of SERS demonstrated by the detection of single molecules, the ability to measure very small samples, and the ability to identify molecular structures of drugs through the rich vibrational information provided by Raman spectroscopy. A further advantage of this detection method is the ease of adding a new detectable analyte by adding its spectral "fingerprint" to the spectral library. In order to allow for drug detection on location, portable Raman spectrometers are needed.

WO 2018/100202 A1 describes a method for detecting an analyte in a sample comprising steps: (a) receiving a sample containing an analyte by means of a sampling device comprising a sample matrix and optionally an eluent for eluting the analyte from the sample matrix, (b) introducing the sampling device into an analysis device comprising at least a first and a second region, wherein the first region is configured for introducing the sampling device and the second region is configured for detecting the analyte, (c) transferring the analyte from the first region to the second region of the analysis device, and (d) determining the presence or/and amount of the analyte in the second region by means of SERS.

WO 2013/006391 A1 describes a method and apparatus for screening drug offenders. A self-contained drug screening apparatus is described, comprising: a breath inlet component for receiving an exhaled air flow of a person, the exhaled air flow including saliva; a sensor for sensing a presence of a drug in the saliva; and an identification module for detecting an identifying characteristic of a person.

WO 2012/051451 A2 describes a highly efficient plasmonic devices, molecule detection systems, and methods of making the same. This publication mentions that SERS substrates described therein could be used in a controlled-substance detection system. The SERS substrate could be used *inter alia* in conjunction with a handheld unit for roadside drug detection applications. It is further suggested the microfluidics, as discussed above, could also be incorporated into a SERS device/slide. Microfluidics would be used to route the material, e.g. saliva. Dilution could be used, mixtures, etc. other standard "Lab-on-chip" methods could be incorporated as are known in the art. WO 2012/051451 A2 further mentions in a very general way that substrates could have multiple areas in combination each addressing a particular analyte by either improving or reducing the binding of one or more species of controlled substances. For example, including a first type of area on the substrate would allow for physicochemical adsorption of an analyte. A second area would provide selective transport molecules based on chemical properties (hydrophobic/hydrophilic, charge, host-guest, molecular imprinting or antibodies to bind to specific biologies). A third area would provide a low resistance path for the bulk fluid access or fluidic pressure systems (passive or active).

WO 2007/078635 A2 describes a method to detect small molecules binding to proteins using SERS. Analytes are said to include drugs, metabolites, pesticides, pollutants, and the like. Morphine, codeine, heroin are mentioned among other drugs. The analyte may be from a body fluid. Sample pretreatment is mentioned. Among body fluids, saliva is listed along with many others.

WO 2007/078635 A2 describes a specific embodiment for detecting the binding of small molecules to proteins in which the proteins are mixed and purified through filtration in a fluid state. This involves a size exclusion spin column and the method suggested makes use of a binding affinity between a small molecule and a protein. The solvent used in the purification column is preferably chosen to optimize binding between the small molecules and the proteins. It is further described that small molecules that do not bind to the proteins are then separated from the proteins by centrifugation in the purification column. It is further mentioned that Raman spectroscopy using SERS active particles could be used to detect the small molecules in the flow through. Thus, according to the method described in this document, Raman spectroscopy is used to detect the presence of the small molecules while they are attached to the proteins.

US 2006/0084181 A1 describes a method and apparatus for rapid extraction and analysis, by SERS, of drugs in saliva. This document mentions that drug species and interfering chemicals may be mutually separated by a chemical treatment, by a physical treatment and/or by a chromatographic method. It further mentions that physical treatment for effecting mutual separation may involve passage of the sample through a filter. Suitable filters would comprise porous substrates such as paper, coated paper, paper fibers, polymer, polymer fibers, mixed paper and polymer fibers, cellulose acetate, glass wool, cotton, diatomite, porous glass, sintered glass, zirconia-stabilized silica, derivatized silica-based matrices, sol-gels, and derivatized sol-gels. They may also comprise a supported membrane covered with separation materials, such as the silica gels, zirconia-stabilized silica, derivatized silica-based matrices, sol-gels, derivatized sol-gels, glass beads, long-chain alkane particles, derivatized long-chain alkane particles, polymers, derivatized polymers, functionalized membranes, alumina, polystyrene, dendrimers, immobilized crown ethers, and ion-exchange resins. It is further stated that chromatographic methods may employ the same separation materials, and will desirably utilize a liquid carrier solvent for at least one of the drug(s) and metabolite(s).

It turned out that the above prior art technologies are not fully satisfying. Common constituents of body liquids still appear to hinder a SERS based reliable quantitative and/or semi-quantitative determination of drugs and their metabolites. "semi-quantitative" in connection with the invention means that the test can be positive or not positive for drug.

The object of the present invention is the provision of a device that enables an efficient and accurate determination of a wide variety of analytes in fluids, particularly of drugs in body fluids, e.g. of illicit drugs in saliva of drivers of motor vehicles in roadside testing but preferably also of substances and drugs on surfaces of goods and transport equipment, such as containers or vehicle interiors. In particular, the device should have a high sensitivity and specificity to a wide variety of analytes, be simple to use and implement, and enable a rapid determination.

The aforesaid object is achieved by the subject-matter of claim 1.

According to the present invention, a device, e.g. a vertical-flow device, for pretreating a sample, e.g. a body fluid sample isolated from a human or a material sample is provided, comprising: a pretreatment zone which is permeable for at least part of a liquid comprised by the sample, wherein the pretreatment zone comprises an organic polymer and/or an inorganic oxide or hydroxide.

Surprisingly, it has been found that applying SERS to samples pretreated by the device according to the invention resulted in strongly improved spectral quality. The detection limit for detecting illicit drugs in body fluids such as saliva has therefore been improved. The device thus enables an accurate determination of a wide variety of analytes, e.g. of illicit drugs in saliva of drivers. The device further enables an efficient determination of drugs as it can be assembled very easily, i.e. with minimum effort and costs. Organic polymers and inorganic oxides or hydroxides suitable for the pretreatment zone are commercially available and can be arranged very easily such that the permeability for a liquid comprised by the body fluid sample is achieved. As the device in essence removes disturbing background signals from spectra detectable in a later SERS step, it is universally applicable for the determination of a broad range of different drugs. It therefore ensures a high sensitivity and specificity to a wide variety of drugs in a later SERS step. It further can be used for saliva which is the body fluid that is most easily accessible and which is minimally invasive for roadside testing.

The device typically has an inlet and an outlet and the liquid permeates the pretreatment zone.

For example, this can be achieved without the need for applying pressure. The device thus can be used by police officers or other roadside test personnel without specific training. This means that the device is simple to implement, and that it enables a rapid determination.

The permeation of the pretreatment zone by the liquid can alternatively be achieved or accelerated by applying pressure, e.g. by connecting the device to a pump or a syringe.

The increase in sensitivity achieved by the invention has so far been achieved only by applying complex and time-consuming separation processes for removing individual disturbing constituents from a biological fluid sample in a laboratory setup. The invention enables a very fast pretreatment followed by subsequent drug detection within a few minutes. This is highly important for Point-of-Care (PoC) testing. According to the prior art, similar drug testing results can be achieved in essence in a laboratory using chemicals that are not even safe for use in PoC testing.

The basic idea underlying the present invention is thus a simple and fast removal of constituents from liquids, such as protein or other components of body fluids. Proteins and other components which have been identified in tests as examples of signal manipulating components are: mucins, amylases and thiocyanate. Such components so far hindered a reliable quantitative or semi-quantitative determination of drugs, e.g. a SERS based reliable quantitative or semi-quantitative determination of drugs. This is inter alia achieved by providing and establishing a pretreatment zone in the device according to the invention.

Although even very small amounts of pure substances and drugs can be detected (for example by SERS), substance and drug detection is several orders of magnitudes less sensitive when the substance or drug is present as a component of a mixture or in a complex matrix. It has now surprisingly been found that a rather simple sample pretreatment according to the invention can be sufficient for inceasing sensitivity by one or even more orders of magnitude when measuring with complex matrices.

According to the invention, the sample can be any sample comprising a liquid. A preferred samples is a body fluid sample isolated from a human. The sample can also be a material sample.

The "body fluid sample" which can be pretreated according to the invention can be diluted or undiluted. It can also be a body fluid sample obtained from an upstream treatment step carried out after the isolation of the body fluid sample from the human but before subjecting the body fluid sample to the pretreatment according to the invention.

The body fluid sample can be a saliva sample, a urine sample, a sweat sample, a blood sample, a plasma sample, a serum sample, a semen sample, a stool sample, a sputum sample, a cerebral spinal fluid sample, a tears sample, a mucus sample, and the like. A preferred body fluid sample is a saliva sample. The saliva sample is most preferably a diluted saliva sample. It can be diluted with an aqueous diluent, e.g. with a buffer.

The body fluid sample is most preferably an aqueous sample. An aqueous sample in that sense contains at least 85 % by weight of water.

"Material sample" in connection with the invention refers to any sample comprising a test material. The test material can, for example, comprise a substance taken from a surface of a good or of a transport equipment, e.g., from a container or vehicle interior. Such surfaces are tested for drugs and other illegal substances, for example in tests performed by customs.

A preferred material sample is a liquid or a dispersion comprising the test material.

The device comprises a pretreatment zone which is permeable for at least part of a liquid comprised by the sample. This means that the pretreatment zone can, for example, comprise interconnected pores or channels. These interconnected pores or channels define flow paths for the liquid to permeate the pretreatment zone. Such pores or channels are present, for example, between the fibers comprised by a glass fiber or nitrocellulose fiber layer or in a sintered polymer layer.

According to the invention, the pretreatment zone comprises an organic polymer and/or an inorganic oxide or hydroxide. The inventors' experimental results indicate that organic polymers and inorganic oxides or hydroxides are particularly useful for removing sample constituents, e.g. body liquid constituents and in particular saliva constituents that appear to be most disturbing in SERS.

As used herein, the expression "inorganic oxide or hydroxide" refers to an inorganic oxide or to an inorganic hydroxide or to a mixed oxide and hydroxide. Many inorganic compounds are mixed oxides and hydroxides. Hydroxyl groups can be present in particular on surfaces. The inorganic oxide or hydroxide is preferably present in a water-insoluble form. Inorganic oxides or hydroxides in water-insoluble forms are very widespread. Many glasses and minerals are examples of inorganic oxides or hydroxides present in a water-insoluble form.

The inorganic oxide or hydroxide can be in any form that enables a permeation of at least part of a liquid comprised by the body fluid sample.

The inorganic oxide or hydroxide can be a mineral fiber, preferably a glass fiber.

Mineral fibers and glass fibers have large surface areas, are inherently immobile (as compared to small particles) and thus fixed in the pretreatment zone. Mineral and glass fibers are commercially available in the form of thin layers, e.g. thin non-woven fiber layers, from which small portions can be punched out for preparing a layered pretreatment zone.

The use of a mineral or preferably glass fiber thus further improves the pretreatment, allows for simple manufacturing of the device and avoids any need of further means for fixing the "inorganic oxide or hydroxide" in the pretreatment zone.

Any polymer surface is capable of binding biopolymers contained in saliva and therefore believed to be useful for a pretreatment according to the invention.

The organic polymer preferably contains carbon-oxygen bonds and/or carbon-halogen bonds wherein halogen is selected from fluorine and chlorine and/or carbon-nitrogen bonds.

Such bonds from carbon to elements having higher electronegativity provide polarity that is assumed to favor an adsorption of mucopolysaccharides and glycoproteins from saliva through polar interactions and/or hydrogen bonding, in particular to carbohydrate residues of the mucopolysaccharides and glycoproteins.

The organic polymer more preferably contains carbon-oxygen bonds and/or carbon-fluorine bonds.

The organic polymer can contain carbohydrate residues. The organic polymer can be a polysaccharide which may be modified or not.

The organic polymer can, for example, be a modified polysaccharide, preferably a modified cellulose, e.g. a nitrocellulose.

The term nitrocellulose covers any degree of nitration and includes nitrocelluloses containing less than one nitro group per glucose monomer to three nitro groups per glucose monomer.

Polysaccharides are particularly rich in polar bonds and have a high density of hydrogen bond donors and/or acceptors. This favors the adsorption of mucopolysaccharides and glycoproteins from saliva even further.

Use of such polymers therefore tends to increase the amount of disturbing body fluid constituent that can be bound per amount of organic polymer. The device thus requires less volume of organic polymer and smaller devices can be used per volume of body fluid sample to be treated.

The organic polymer can be an organic polymer fiber.

Organic polymer fibers have large surface areas, are inherently immobile (as compared to small particles) and thus fixed in the pretreatment zone. Organic polymer fibers are commercially available in the form of thin layers, e.g. thin non-woven fiber layers, from which small portions can be punched out for preparing a layered pretreatment zone.

The use of organic polymer fibers thus further improves the pretreatment, allows for simple manufacturing of the device and avoids any need of further means for fixing the organic polymer in the pretreatment zone.

A preferred organic polymer fiber is a nitrocellulose fiber.

The pretreatment zone preferably comprises at least two layers, preferably at least three layers, for example three to six layers, wherein at least one layer comprises the organic polymer, e.g. the organic polymer fiber, and wherein at least another layer comprises the inorganic oxide or hydroxide, e.g. the mineral fiber.

Within the scope of the invention, it is also possible to use a layer containing the organic polymer and the inorganic oxide or hydroxide. An example of a layer comprising the organic polymer and the inorganic oxide or hydroxide is a mixed non-woven layer comprising the organic polymer fiber and the mineral fiber, e.g. the nitrocellulose fiber and the glass fiber.

In a particularly preferred device according to the invention, the pretreatment zone comprises a glass fiber layer and/or a nitrocellulose fiber layer, e.g. a glass fiber layer and a nitrocellulose fiber layer.

Alternatively or in addition, the pretreatment zone may comprise a porous sintered material. Specific examples of porous sintered materials are porous sintered polymer materials, in particular porous sintered polyethylene or porous sintered polypropylene having an average pore size in a range from 10 to 150 µm.

The inorganic oxide or hydroxide can also be a sintered material containing the inorganic oxide or hydroxide.

The pretreatment zone can, for example, comprises at least two, preferably at least three, more preferably at least four, for example three to eight or four to six fiber layers.

The expression "fiber layer" means that the layer contains fibers. It does not exclude other non-fiber layer constituents.

Any layer can be a composite layer. A preferred composite layer can comprise the organic polymer and the inorganic oxide or hydroxide. A preferred fiber layer, for example, can comprise different fibers, e.g. a glass fiber and a nitrocellulose fiber.

Such integration of both materials, i.e. of the inorganic oxide or hydroxide and the organic polymer (e.g. of the glass fiber and the nitrocellulose fiber) into a single layer may avoid any need for selectively arranging multiple layers on top of each other. This avoids a source of error in the assembly of the device of the invention and therefore may allow for more reliable pretreatment results.

The invention further does not exclude that different layers are glued onto one another.

The pretreatment zone may comprise a layer or several layers which specifically bind(s), cleaves, or precipitates a defined constituent or several defined constituents of the sample. Specific binding may involve chemical bonding by immobilized reactive groups or specific binding by protein-ligand or protein-protein interaction, e.g. immunologic binding. Specific cleavage may involve an immobilized protease specific for a protein comprised by the sample.

Preferably at least one fiber layer is a woven or non-woven fiber layer.

A particularly preferred pretreatment zone contains at least three, preferably at least four, e.g. at least five layers, wherein at least one nitrocellulose fiber layer is sandwiched between two glass fiber layers. Preferably, the order of layers can be:
- glass fiber layer
- nitrocellulose fiber layer
- glass fiber layer
- nitrocellulose fiber layer
- glass fiber layer.

The pretreatment zone and the organic polymer and/or an inorganic oxide or hydroxide comprised by the pretreatment zone can be selected and configured such that at least part of a liquid (preferably aqueous liquid) comprised by the body fluid sample (preferably aqueous body fluid sample) can pass through the pretreatment zone by capillary forces acting on the liquid when the liquid passes through the pretreatment zone.

This can be highly favorable as no pump or similar device is required for using the device of the invention. However, the use of pumps, syringes or microfluidic elements is not excluded by the intention.

The pretreatment zone and the organic polymer and/or an inorganic oxide or hydroxide comprised by the pretreatment zone can therefore also be selected and configured such that a liquid (preferably aqueous liquid) comprised by the body fluid sample (preferably aqueous body fluid sample) cannot pass through the pretreatment zone only by capillary forces acting on the liquid when the liquid passes through the pretreatment zone.

The pretreatment zone can comprise an immobilized SERS active substrate. This is advantageous as the device can be used without an additional step of adding a SERS active substrate. A more widespread use of the device will be possible. Using the device will require even less skills.

The pretreatment zone can preferably be arranged in a tube. One of the ends of the tube can serve as an inlet for directing the sample to the pretreatment zone. The other end of the tube can serve as an outlet.

This is advantageous as a multiple layer pretreatment zones can be provided in the tube by simply pressing circular disks of layer material in the tube onto one another.

Alternatively, the pretreatment zone can be sandwiched between an inlet element and an outlet element, wherein the inlet element comprises an inlet and the outlet element comprises an outlet.

At least one of the inlet element and outlet element can be a plate. The shape and dimensions of the plate surface can be the same as the shape and dimensions of a layer comprised by the pretreatment zone. The walls of the device can then be provided by a hollow cylindric element which has inner dimensions matching with the dimensions of the plate surface and with the dimensions of the layer.

This is advantageous as the device can then be made very easily by placing the layer (and optionally other layers) in the hollow cylindric element and putting the inlet element and/or outlet element as outermost element(s) in the hollow cylindric element on the layered stack formed therein.

The other inlet element or outlet element can also be a plate. The other element or outlet element can then form the opposite outermost element in the hollow cylindric element below the layered stack formed therein. Alternatively, the other inlet element or outlet element can be an integral preformed bottom part (with the inlet or outlet) of the hollow cylindric element.

Preferably, a hollow cylindric element defines a channel from the inlet to the outlet. The hollow cylindric element can, for example, be a circular or an angular hollow cylindric element. The angular hollow cylindrical element can, for example have a rectangular base shape, a squared base shape or a hexagonal base shape. It can be the tube as mentioned herein.

The total volume of the pretreatment zone is preferably less than 10 cm³, particularly less than 6 cm³, e.g. less than 4 cm³.

The invention further relates to a method for pretreating a sample, e.g. a body fluid sample from a human and/or a material sample, wherein the sample is brought into contact with a pretreatment zone which is permeable for at least part of a liquid comprised by the sample, wherein the pretreatment zone comprises an organic polymer and/or an inorganic oxide or hydroxide.

The body fluid sample from the human is preferably a saliva sample from the human.

The saliva sample can be diluted or undiluted. It can also be a saliva sample obtained from an upstream treatment step carried out after the isolation of the body fluid sample from the human but before carrying out the pretreatment of the saliva sample according to the method of the invention.

The saliva sample is most preferably a diluted saliva sample. It can be diluted with an aqueous diluent, e.g. with a buffer.

Preferably, at least part of a liquid comprised by the sample is passed through the pretreatment zone.

Preferably, the method for pretreating the sample is carried out in a device according to the invention.

According to a preferred method of the invention, the sample, e.g. body fluid sample or saliva sample can, for example, be fed into the device via the inlet and at least part of the liquid comprised by the body fluid sample is passed through the pretreatment zone to the outlet.

Optionally, at least part of a liquid comprised by the sample, e.g. the body fluid sample can be passed through the pretreatment zone by capillary forces acting on the liquid when the liquid passes through the pretreatment zone.

The method for pretreating a body fluid sample as described herein provides a pretreated sample. The pretreated sample can be analyzed for drugs contained therein.

The invention further relates to a method for drug testing, e.g. roadside drug testing, wherein a body fluid sample from a human, e.g. a saliva sample from a human, is pretreated according to the method for pretreating a body fluid sample described herein and the pretreated sample is analyzed for drugs contained in the pretreated sample.

The pretreated sample is preferably analyzed for drugs contained in the pretreated sample by spectroscopy. The spectroscopy is preferably Raman spectroscopy, most preferably SERS.

Preferably, a liquid having passed the pretreatment zone is brought into contact with a SERS active substrate and the liquid is then analyzed for drugs contained therein by SERS.

The SERS active substrate can be added to the pretreated sample. The SERS active substrate can, for example, be brought into contact with the liquid which has reached the end of the pretreatment zone.

Alternatively, the pretreatment zone can comprise an immobilized SERS active substrate. This is advantageous as it avoids the need for an additional step of adding the SERS active substrate. A more widespread use of the method of the invention even by less skilled personnel will be possible.

The invention also relates to the use of a material capable of binding at least one of thiocyanate and biopolymers contained in human saliva for pretreating saliva from a human for drug testing.

The invention further relates to the of an organic polymer and/or an inorganic oxide or hydroxide for pretreating saliva from a human for drug testing.

The devices, methods, and uses as described herein are different aspects of the same invention. Any feature, effect or advantage described in connection with one of these aspects applies also for the other aspects.

The invention will be described in greater detail by way of the following non-limiting drawings and non-limiting examples.

In the figures it is shown:
- **Fig. 1**: a schematic basic structure of a first device according to the invention;
- **Fig. 2**: a schematic basic structure of a second device according to the invention;
- **Fig. 3**: an inlet element in two different views;
- **Fig. 4**: the inlet element of **Fig. 3** in a different view;
- **Fig. 5**: a scheme illustrating a method according to the invention;
- **Fig. 6-10**: Raman spectra detected by SERS (horizontal axes: raman shift [cm⁻¹] vertical axes: signal relative intensity);
- **Fig. 11**: a concentration dependency of the signal-to-noise achieved by the invention; and
- **Fig. 12**: a pretreatment zone arranged in a tube.

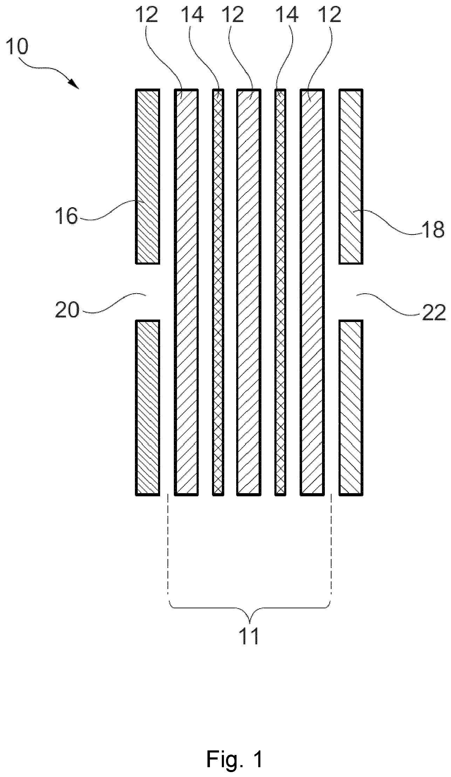

**Fig. 1** shows as possible embodiment of the present invention a first device 10 for pretreating a body fluid sample isolated from a human.

The device comprises a pretreatment zone 11.

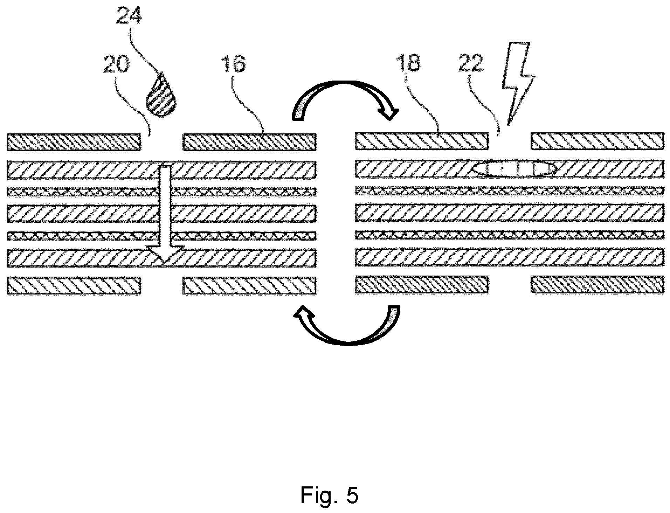

As explained with regard to **Fig. 5** below, the pretreatment zone 11 is permeable for a liquid comprised by a body fluid sample 24.

The pretreatment zone comprises an organic polymer and an inorganic oxide or hydroxide.

In this first device, the inorganic oxide or hydroxide is a glass fiber.

The organic polymer is a nitrocellulose fiber.

The pretreatment zone 11 comprises five layers 12, 14, wherein two layers 14 comprise the nitrocellulose fiber, and three other layers 12 comprises the glass fiber.

All layers 12, 14 are non-woven fiber layers.

The pretreatment zone 11 is sandwiched between an inlet element 16 and an outlet element 18.

The inlet element 16 comprises an inlet 20.

The outlet element 18 comprises an outlet 22.

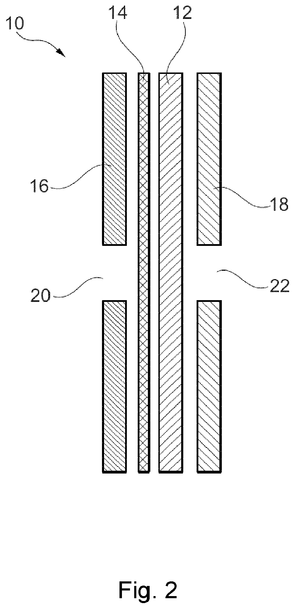

**Fig. 2** shows a second device 10. The second device 10 differs from the first device in that the number of layers is reduced to only one non-woven glass fiber layer 12 and one non-woven nitrocellulose fiber layer 14.

In **Fig.** 1 and **Fig. 2****,** the distances between the layers 12, 14, and from the layers to the inlet and outlet elements 16, 18, are for illustrative purpose only.

In devices according to the invention, layers are typically placed directly on one another without any distance.

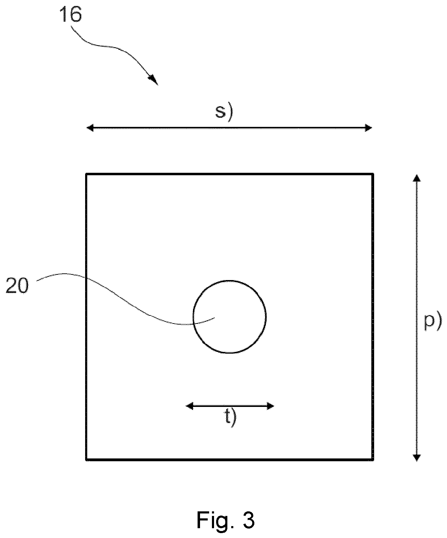

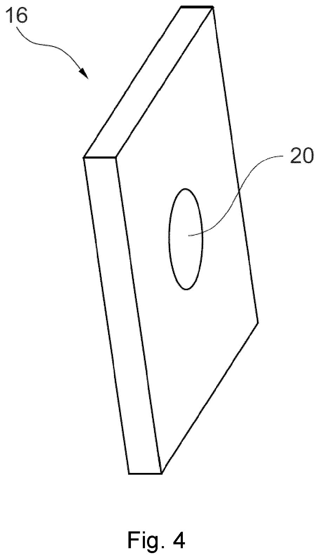

**Fig. 3** and **Fig. 4** show an inlet element 16 from **Fig.1** and **Fig. 2** in two different views.

The inlet element 16 is a squared plate, i.e. lengths r) and s) are each 20 mm.

The inlet is a through-hole extending from one surface of the inlet element to the other surface of the inlet element.

The diameter t) of the inlet is 5 mm.

The thickness of the inlet element is 0.5 mm.

A second inlet element 16 can be used as an outlet element 18 in the devices shown in **Fig. 1** and **Fig. 2****.**

The scheme in **Fig. 5** illustrates a method according to the invention for drug testing. The method is carried out in a device 10 as shown in **Fig. 1****.**

As shown in Figure 5, a body fluid sample 24 which contains saliva from a human is fed into inlet 20 of inlet element 16 and brought into contact with the pretreatment zone.

The pretreatment zone is permeable for at least part of a liquid comprised by the body fluid sample and the liquid passes through the pretreatment zone by capillary forces acting on the liquid.

Pretreated sample thus accumulates in outlet 22 and/or in the non-woven glass fiber layer 12 closest to the outlet 22.

The device is then turned around and the pretreated sample which has accumulated in outlet 20 and/or in the non-woven glass fiber layer 12 closest to the outlet 22 is analyzed for drugs contained in the pretreated sample by SERS.

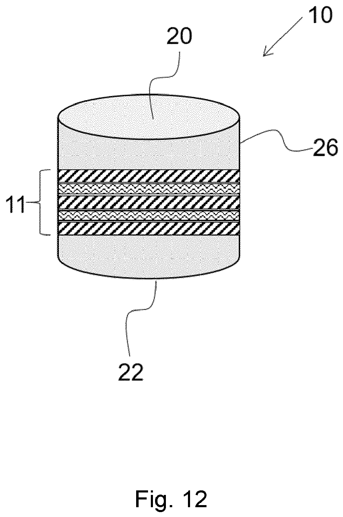

**Fig. 12** shows another device 10 according to the invention. A multiple layer pretreatment zone 11 is arranged in a tube 26. One of the ends of the tube serves as an inlet 20 for directing the sample to the pretreatment zone. The other end of the tube can serve as an outlet 22.

### Example 1

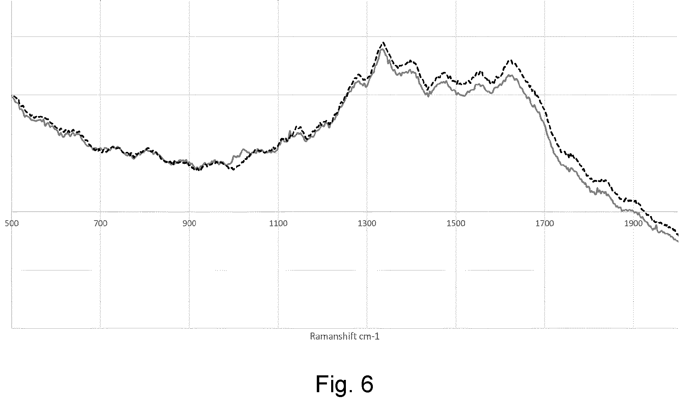

SERS was applied for recording spectra of methamphetamine in different aqueous media The spectra are shown in **Fig. 6**

| | |
|---|---|
| line: | methamphetamine in saliva (2500 ng/ml) without pretreatment |
| dashed line: | saliva without pretreatment |

This example demonstrates a strong impact of saliva constituents on the determination of drugs. Without pretreatment of saliva, the spectrum of saliva with drug is in essence undistinguishable from the spectrum of saliva without drug.

### Example 2

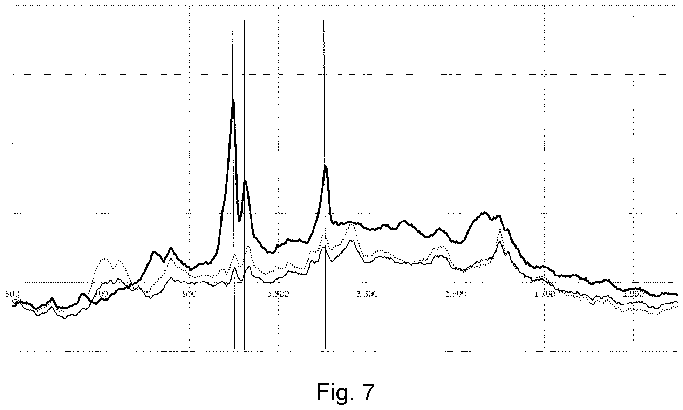

SERS was applied for recording spectra of methamphetamine in different aqueous media. The spectra are shown in **Fig. 7**

| | |
|---|---|
| bold line: | methamphetamine in water (2000 ng /ml) |
| thin line: | methamphetamine in saliva (2000 ng/ml) |
| dotted line: | saliva |

SERS was applied in each case after a pretreatment in a device as shown in Fig. 2.

This example demonstrates that a pretreatment of a saliva sample by a pretreatment zones of Fig. 2 results in a significant improvement of spectral quality.

A strong reduction of disturbing background saliva signals is observed, which is due to the pretreatment according to the invention. While methamphetamine related signals fully disappeared in the background saliva signals in the spectra of **Fig. 6****,** the pretreatment in a device according to **Fig. 2** of the invention resulted in a partial removal of these disturbing signals and in the appearance of characteristic drug signals.

However, this improvement in spectral quality will be sufficient only for identifying drug abuse in particularly high doses. A treatment in a device according to **Fig. 2** thus is useful as a pretreatment but not always sufficient for an accurate semi-quantitative drug detection, when used as such, i.e. without further pretreatment. Nevertheless, it can provide at least an important contribution to sample pretreatment.

### Example 3

The ability of different layers for binding proteins was studied by staining the protein comprised by a buffer, and subjecting the buffer to single-layer and multilayer test setups. This demonstrated that a glass fiber layer and a nitrocellulose fiber layers is capable of binding significant amounts of protein but not all of the protein subjected to the layers. This finding was consistent with the results of example 1 and indicated that protein constituents of saliva could have contributed to the strong loss in signal intensity observed in example 1.

### Example 4

Four solutions were prepared for testing the impact of thiocyanate as one example of a disturbing sample constituent.

| | |
|---|---|
| Solution 1: | aqueous solution of methamphetamine (2000 ng/ml) |
| Solution 2: | aqueous solution of methamphetamine (2000 ng/ml) and thiocyanate (10 µM) |
| Solution 3: | aqueous solution of methamphetamine (2000 ng/ml) and thiocyanate (1 mM) |
| Solution 4: | aqueous solution of methamphetamine (2000 ng/ml) and thiocyanate (1 M) |

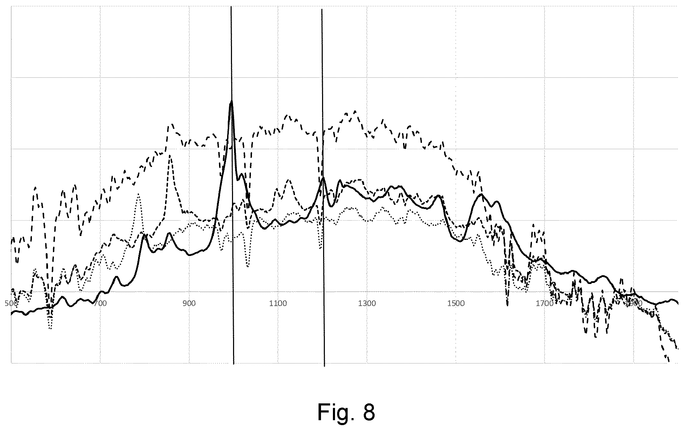

Each of the solutions was passed through a device according to Fig. 2 and analyzed by SERS. The spectra are shown in **Fig. 8**

| | |
|---|---|
| line: | SERS of solution 1 |
| dashed line with large spaces between the line segments: | SERS of solution 2 |
| dashed line with small spaces between the line segments: | SERS of solution 3 |
| dotted line: | SERS of solution 4 |

### Example 5

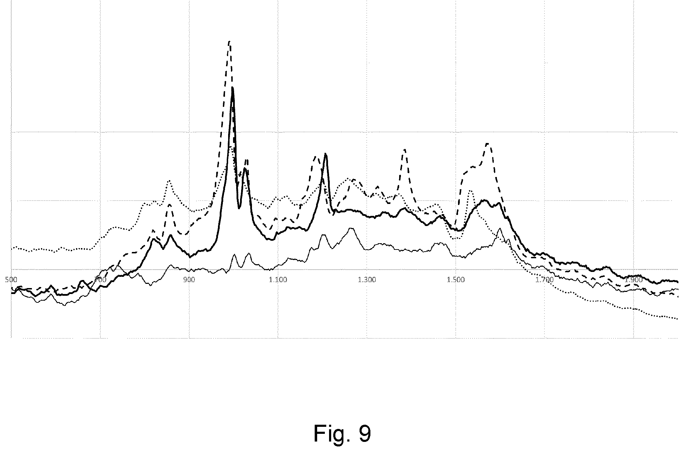

Two samples of saliva containing methamphetamine (2000 ng/ml) were pretreated and analyzed by SERS according to the method shown in **Fig. 5** (dashed and dotted line in **Fig. 9**) The layers comprised by the pretreatment zone were two glass fiber layers followed by one nitrocellulose fiber layer.

A reference sample was pretreated and analyzed in the same way. The spectrum is shown in **Fig. 9** (bold line: SERS of pretreated water containing methamphetamine (2000 ng/ml))

The dashed and dotted lines in **Fig. 9** resemble the bold line. This clearly shows that disturbing sample constituents that would otherwise overlap with drug signals have been removed by the pretreatment. Example 5 thus demonstrates that drugs can be determined very accurately from saliva samples when the pretreatment is carried out through a pretreatment zone comprising two glass fiber layers and one nitrocellulose fiber layer.

### Example 6

Saliva containing methamphetamine (2000 ng/ml) was pretreated and analyzed by SERS according to the method shown in **Fig. 5****.** The layers comprised by the pretreatment zone were as shown in **Fig. 1** and **Fig. 5****.** Two reference samples of saliva without methamphetamine were pretreated and analyzed in the same way.

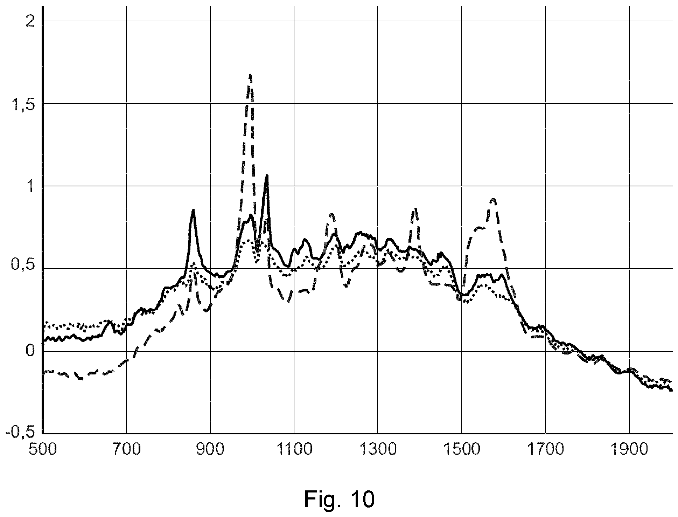

The spectra are shown in **Fig. 10****.**

| | |
|---|---|
| dashed line: | SERS of pretreated saliva sample containing methamphetamine |
| line and dotted line: | SERS of pretreated saliva reference sample without methamphetamine |

The spectra in **Fig. 10** demonstrate that drugs can be determined very accurately from saliva samples when the pretreatment is carried out through a pretreatment zone of **Fig. 1****.**

The invention is thus not only suitable for identifying drug abuse when drugs are highly dosed. More advanced (e.g. multi-layer) pretreatment zones allow for a highly efficient pretreatment of body fluids that extends the scope of the invention also to identifying drug abuse at lower dosage.

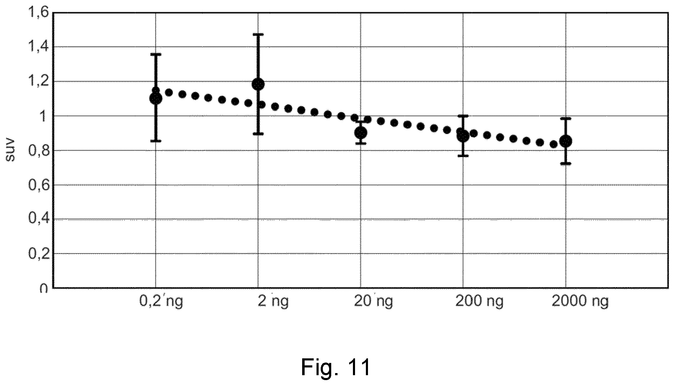

Further tests with multiple samples at a broad range of different concentrations (results and standard deviations are indicated in **Fig. 11**) indicate that methamphetamine can be identified even when samples contain only single digit to two digit ng/ml amounts of methamphetamine, at least when samples do not even contain 20 ng of methamphetamine per milliliter.

The interaction with body fluid constituents is not expected to be limited to organic polymer and/or inorganic oxide or hydroxide which is present in the form of fibers. This is because the punctual interaction of a body fluid constituent with a surface of a material comprised by the pretreatment zone does typically not depend on the shape of the material surface adjacent to the interaction site. The claims are thus a reasonable generalization to other materials and shapes of materials than those actually used in the examples.

### Reference signs

- 10: device
- 11: pretreatment zone
- 12, 14: layers
- 16: inlet element
- 18: outlet element
- 20: inlet
- 22: outlet
- 24: body fluid sample
- 26: tube

## Claims

1. A device (10) for pretreating a sample, e.g. a body fluid sample isolated from a human and/or a material sample,
comprising:
a pretreatment zone (11) which is permeable for at least part of a liquid comprised by the sample,
wherein the pretreatment zone comprises
an organic polymer and/or
an inorganic oxide or hydroxide.

2. The device (10) according to claim 1,
**characterized in that**
the inorganic oxide or hydroxide is a mineral fiber, preferably a glass fiber.

3. The device (10) according to claim 1 or 2,
**characterized in that**
the organic polymer contains carbon-oxygen bonds and/or carbon-halogen bonds wherein halogen is selected from fluorine and chlorine and/or carbon-nitrogen bonds.

4. The device (10) according to any of the preceding claims,
**characterized in that**
the organic polymer is a modified polysaccharide, preferably a modified cellulose, e.g. a nitrocellulose.

5. The device (10) according to any of the preceding claims,
**characterized in that**
the organic polymer is an organic polymer fiber, e.g. a nitrocellulose fiber.

6. The device (10) according to any of the preceding claims,
**characterized in that**
the pretreatment zone (11) comprises at least two layers (12, 14), wherein at least one layer (14) comprises the organic polymer, e.g. the organic polymer fiber, and
wherein at least another layer (12) comprises the inorganic oxide or hydroxide, e.g. the mineral fiber.

7. The device (10) according to any of the preceding claims,
**characterized in that**
the pretreatment zone (11) comprises a glass fiber layer (12) and/or a nitrocellulose fiber layer (14).

8. The device (10) according to any of the preceding claims,
**characterized in that**
the pretreatment zone (11) comprises at least two, preferably at least three fiber layers (12, 14).

9. The device (10) according to any of the preceding claims,
**characterized in that**
the pretreatment zone (11) is arranged in a tube (26).

10. The device (10) according to any of the preceding claims,
**characterized in that**
the pretreatment zone (11) is sandwiched between an inlet element (16) and an outlet element (18), wherein the inlet element (16) comprises an inlet (20) and the outlet element (18) comprises an outlet (22).

11. A method for pretreating a sample, preferably a body fluid sample from a human and/or a material sample, e.g. a saliva sample from a human,
wherein the sample is brought into contact with a pretreatment zone (11) which is permeable for at least part of a liquid comprised by the sample,
wherein the pretreatment zone comprises
an organic polymer and/or
an inorganic oxide or hydroxide.

12. The method according to claim 11,
**characterized in that**
at least part of a liquid comprised by the body fluid sample is passed through the pretreatment zone (11).

13. A method for drug testing, wherein a body fluid sample from a human, e.g. a saliva sample from a human, is pretreated according to the method of claim 11 or 12 and the pretreated sample is analyzed for drugs contained in the pretreated sample.

14. Use of a material capable of binding at least one of thiocyanate and biopolymers contained in human saliva for pretreating saliva from a human for drug testing.

15. Use of an organic polymer and/or an inorganic oxide or hydroxide for pretreating saliva from a human for drug testing.
